# EUROPÄISCHE PATENTSCHRIFT

(11) **EP 0 949 239 B1**
(45) Veröffentlichungstag und Bekanntmachung des Hinweises auf die Patenterteilung: **08.01.2003**
(21) Anmeldenummer: 99106027.8
(22) Anmeldetag: 25.03.1999
(51) Int. Cl.: C07C 67/46, C07C 69/145

(54) **Verfahren zur Herstellung von Linalylacetat**
Process for the preparation of linalyl acetate
Procédé de préparation de l'acétate de linalyle

(30) Priorität: 09.04.1998 DE 19815833
(43) Veröffentlichungstag der Anmeldung: 13.10.1999
(73) Patentinhaber: BASF AKTIENGESELLSCHAFT, 67056 Ludwigshafen (DE)
(72) Erfinder: Aquila, Werner, Dr., 68309 Mannheim (DE); Pox, Roland, 67059 Ludwigshafen (DE); Etzrodt, Heinz, Dr., 67434 Neustadt (DE)

(56) Entgegenhaltungen:
- DE-B- 1 643 714

## Beschreibung

Die Erfindung betrifft ein verbessertes Verfahren zur Herstellung von Essigsäureestern von Alkoholen und Phenolen, insbesondere von Linalylacetat durch Umsetzen mit Keten in Gegenwart von Katalysatoren.

Die Ester von Monocarbonsäuren, wie der Essigsäure, sind im allgemeinen wohlriechende Verbindungen, die u.a. in ätherischen Ölen vorkommen. Wegen ihrer olfaktorischen Eigenschaften haben viele Ester höherer Alkohole Bedeutung in der Riechstoffindustrie erlangt. Dies gilt insbesondere für 3,7-Dimethyl-1,6-octadien-3-yl-acetat (Linalylacetat) der Formel I 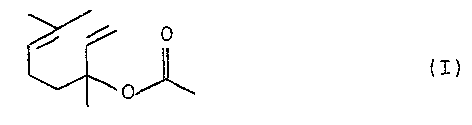 mit seinem frisch-blumigen, an Bergamottöl erinnernden Duft.

Es hat daher nicht an Versuchen gefehlt, günstige Verfahren zur Herstellung von Essigsäureestern auch in industriellem Maßstab zu finden. Die bekanntesten Verfahren zur Herstellung von Estern höherer Alkohole sind die Umsetzung der Alkohole mit Carbonsäurehalogeniden oder mit Carbonsäureanhydriden. Nachteilig bei der Umsetzung mit Carbonsäurehalogeniden ist, daß bei deren Umsetzung Halogenwasserstoffsäuren gebildet werden, die allgemein zu Korrosionsproblemen führen und bei tertiären Alkoholen Wasserabspaltungen und dadurch vielfach Polymerisationen verursachen.

Bei der Umsetzung mit Carbonsäureanhydriden besteht der Nachteil, daß im Reaktionsgemisch äquimolare Mengen der entsprechenden Carbonsäure gebildet werden, die das Gleichgewicht bei der Veresterung ungünstig beeinflussen und deshalb ständig aus dem Reaktionsgemisch entfernt werden müssen, was technisch recht aufwendig ist.

Für die Herstellung von Essigsäureestern ist außerdem die Umsetzung von hydroxylgruppenhaltigen Verbindungen mit Keten in Gegenwart von Katalysatoren bekannt. Gemäß Angewandte Chemie, Bd. 68 [1956], S. 361 bis 363 verlaufen Acylierungen mit Keten jedoch insbesondere bei höheren Alkoholen nur unvollständig, da das entstehende Acylierungsprodukt eine Hemmung der weiteren Reaktion bewirkt. Nach der genannten Literaturstelle werden als Katalysatoren vorwiegend Lewissäuren, wie Schwefelsäure, p-Toluolsulfonsäure, Phosphorsäure, Bortrifluorid, Bortrifluorid-Ätherat oder Kaliumhydrogensulfat verwendet. Da diese Katalysatoren jedoch starke Säuren sind oder, wie im Falle des Bortrifluorids, in Gegenwart von Spuren von Wasser stark saure Folgeprodukte liefern, können sie in Metallapparaturen Korrosion hervorrufen. Außerdem bewirken die sauren Katalysatoren in manchen Fällen die Bildung von harzartigen Produkten (vgl. The Journal of General Chemistry of the U.S.S.R., Bd. 21 [1951], S. 1147).

Ein weiterer Nachteil dieser Katalysatoren besteht darin, daß die entstandenen Ester von den Katalysatoren nur durch Destillation sauber abgetrennt werden können. Diesen Nachteil weisen auch basische Katalysatoren, wie Harnstoff und spezielle Salze der Essigsäure, auf, deren Verwendung als Katalysatoren für Acetylierungen von hydroxylgruppenhaltigen Verbindungen mit Keten in Angewandte Chemie, Bd. 68 (1956), S. 363, beschrieben wird.

Es ist weiter bekannt, Alkohole mit Keten in Gegenwart eines Sulfonsäuregruppen enthaltenden Ionenaustauschers zu acetylieren (vgl. japanische Patentanmeldung 8334/65). Jedoch haben auch die Ionenaustauscher den Nachteil, daß sie von den Ausgangsstoffen teilweise gelöst werden können. Die Ausbeuten bei diesem Verfahren betragen z.B. bei der Umsetzung von Linalool zu Linalylacetat nur etwa 83 %.

Zur Überwindung der Nachteile des vorgenannten Standes der Technik wird gemäß DE-OS 1 643 712 die Umsetzung von Alkoholen mit Keten in Gegenwart von Aluminiumsilikaten mit austauschbaren Kationen empfohlen. Dies Verfahren, das für die Herstellung von Essigsäureestern primärer Alkohole vorteilhaft ist, hat für die Herstellung von Essigsäureestern tertiärer Alkohole, wie dem Linalylacetat, den großen Nachteil, daß trotz Verwendung sehr großer Mengen eines speziellen hochaktiven Katalysators (gemäß DE-OS 1 643 712 wird der Katalysator in Mengen von 3 bis 800 Gew.-%, bezogen auf den Alkohol eingesetzt) nur unbefriedigende Umsätze erzielt werden.

Weiterhin ist aus Perfum. Essent. Oil Record 58(12) (1967), Seiten 872-78 bekannt, daß man Linalylacetat durch Einleiten von Keten in Linalool bei 20 bis 30°C herstellen kann, wenn man 0,2 Gew.-% p-Toluolsulfonsäure, 0,3 Gew.-% Phosphorsäure oder 5 Gew.-% eines sauren Kationenaustauscherharzes als Katalysator verwendet. Nachteilig an diesem an sich guten Verfahren ist, daß die Ausbeuten an reinem, für olfaktorische Zwecke geeigneten Linalylacetat nur etwa 80 bis 85 % betragen, und daß nur sehr geringe Raum-Zeit-Ausbeuten erzielt werden. So werden beispielsweise für die Umsetzung von 55 g Linalool Reaktionszeiten von 4 bis 5 Stunden benötigt.

Es war daher die Aufgabe der Erfindung, ein Verfahren zur Herstellung von Linalylacetat durch Umsetzen von Linalool mit Keten in Gegenwart von Katalysatoren bereitzustellen, das es erlaubt, Linalylacetat auch in industriellem Maßstab auf vorteilhafte Weise in sehr guten Ausbeuten, Raum-Zeit-Ausbeuten, und Reinheiten und unter Vermeidung der Nachteile der bekannten Verfahren herzustellen. Dieses neue Verfahren sollte zudem möglichst universell auch auf andere Hydroxylgruppen enthaltende organische Verbindungen anwendbar sein.

DE-B 16 43 714 beschreibt die Herstellung von Essigsäureestern durch Zn-katalysierte Umsetzung von Keton mit verschiedenen Alkoholen unter Zugabe von Zinkoxid.

Gegenstand der Erfindung ist dementsprechend ein Verfahren zur Herstellung von Essigsäureestern durch Umsetzen von eine oder mehrere Hydroxylgruppen enthaltenden organischen Verbindungen mit Keten in Gegenwart von Katalysatoren, das dadurch gekennzeichnet ist, daß man die Umsetzung unter Verwendung von etwa der Anzahl der Hydroxylgruppen in der Hydroxylgruppen enthaltenden Verbindung entsprechenden molaren Menge an Keten und unter intensiver Vermischung des Reaktionsgemisches durchführt und dass man als Katalysatoren Zinksalze von C₂-C₁₈-Carbonsäuren einsetzt, insbesondere ein Verfahren zur Herstellung von Linalylacetat durch Umsetzen von Linalool mit Keten in Gegenwart von Katalysatoren, das dadurch gekennzeichnet ist, daß man die Umsetzung unter Verwendung von etwa äquimolaren Mengen an Linalool und Keten in Gegenwart von Zinksalzen von Carbonsäuren mit 2 bis 18 C-Atomen und unter intensiver Vermischung des Reaktionsgemisches durchführt.

Genannt seien beispielsweise Zinkacetat und Zinkstearat, insbesondere das relativ leicht zugängliche Zinkacetat. Prinzipiell sind auch Zinksalze von Dicarbonsäuren, wie Bernsteinsäure und Oxalsäure oder von Aminocarbonsäuren, wie Alanin oder Leucin einsetzbar, jedoch bringt dies keine weiteren Vorteile.

Sehr vorteilhaft an dem erfindungsgemäßen Verfahren ist auch, daß man die Katalysatoren nur in sehr kleinen Mengen einsetzen muß, was das Verfahren kostengünstiger macht und die Aufarbeitung des Reaktionsgemisches erleichtert. Im allgemeinen verwendet man die Zinksalze in Mengen von 0,1 bis 1,0, vorzugsweise 0,2 bis 0,5, Gew.-%, bezogen auf den eingesetzten Alkohol.

Es war sehr überraschend, daß die beanspruchten Zinkverbindungen so vorteilhaft als Katalysatoren eingesetzt werden können, da man z.B. mit Natriumacetat, welches als Katalysator für Umsetzungen mit Keten bekannt ist, nur völlig unzureichende Ausbeuten erzielt (s. Vergleichsbeispiel 1) und die Acetate von Metallen, wie Silber, Aluminium, Magnesium, Calcium, Mangan (II), Kobalt, Nickel oder Eisen (II) die Umsetzung praktisch überhaupt nicht katalysieren (s. Vergleichsbeispiele 2 bis 9).

Da überschüssiges Keten zu zahllosen Nebenreaktionen führen kann, gelingt das erfindungsgemäße Verfahren nur mit guten Ausbeuten, wenn man die Umsetzung unter Verwendung von nur etwa äquimolaren Mengen an Keten durchführt, da Linalool bei dieser Umsetzung zu Nebenreaktionen neigt.

Da Keten eine äußerst reaktive Verbindung ist, die stark zu Dimerisierung unter Bildung von Diketen neigt, verwendet man im allgemeinen Keten, welches erst kurz vorher hergestellt worden ist. Für die technische Herstellung von Keten gibt es in der Praxis im wesentlichen 2 Wege: die thermische Spaltung von Aceton sowie die thermische Spaltung von Essigsäure.

Das erfindungsgemäße Verfahren gestaltet sich besonders vorteilhaft, wenn man Keten verwendet, welches unmittelbar vor der erfindungsgemäßen Umsetzung durch thermische Spaltung von Essigsäure bei Temperaturen von 800 bis 900, vorzugsweise etwa 850 bis 870°C, und vermindertem Druck von etwa 700 bis 900 mbar, vorzugsweise 750 bis 850 mbar, erhalten worden ist.

Um das so erhaltene Keten auf einfache Weise in das Reaktionsgefäß für die erfindungsgemäße Umsetzung einzubringen, ist es auch für diese Umsetzung von Vorteil, sie unter vermindertem Druck durchzuführen.

Im allgemeinen wird die erfindungsgemäße Umsetzung bei Temperaturen von etwa 35 bis 110°C, vorzugsweise 70 bis 100°C, insbesondere 85 bis 95°C und verminderten Drucken von etwa 400 bis 900 mbar, vorzugsweise 500 bis 600 mbar, durchgeführt.

Es war überraschend, daß die Umsetzung bei so hohen Temperaturen mit Ausbeuten über 90 % an reinem Linalylacetat gelingt, da, in der obengenannten Literaturstelle Perfumery and essential Oil record (London) 58 (12) (1967) auf Seite 875, linke Spalte, Zeile 22 bis rechte Spalte, Zeile 5, ausgeführt wird, daß die Bildung von Nebenprodukten stark ansteigt, wenn man die Temperatur auf 50°C ansteigen läßt und daß bei Temperaturen von 50°C die Ausbeuten an Linalylacetat auf etwa 27 % der Theorie sinken.

Die Reaktionszeiten bei dem erfindungsgemäßen Verfahren sind wesentlich geringer als bei dem letztgenannten Verfahren des Standes der Technik. Während gemäß dem in Perfumery and Essential Oil Record 58 (12) beschriebenen Verfahren für die Umsetzung von 55 g Linalool 4,5 bis 5 Stunden Reaktionszeit notwendig sind, ist es gemäß dem erfindungsgemäßen Verfahren möglich, etwa 1300 kg Linalool in nur etwa 3 Stunden zu Linalylacetat umzusetzen (vgl. Beispiel 4).

Das erfindungsgemäße Acetylierungsverfahren ist universell auf organische Verbindungen, die eine oder mehrere Hydroxylgruppen enthalten, anwendbar. Als geeignete Hydroxylgruppen enthaltende organische Verbindungen seien vor allem einwertige gesättigte und ungesättigte aliphatische, aromatisch-aliphatische oder cycloaliphatisch-aliphatische Alkohole mit 5 bis 20 C-Atomen genannt. Besondere Bedeutung besitzt das Verfahren für die im allgemeinen schwieriger zu veresternden tertiären gesättigten oder ungesättigten aliphatischen, aromatisch-aliphatischen oder cycloaliphatisch-aliphatischen Alkohole mit 5 bis 20 C-Atomen.

Aber auch organische Verbindungen mit phenolischen Hydroxylgruppen, wie das α-Tocopherol, können mit Hilfe des erfindungsgemäßen Verfahrens vorteilhaft acetyliert werden.

Als besonders geeignete primäre Alkohole seien genannt: 3-Methyl-2-buten-1-ol (Prenol), 4-tert-Butyl-cyclohexyl-2-ethanol, 3-tert-Butyl-4-methoxy-cyclohexylmethanol, 2-Cyclododecyl-propanol (Hydroxyambran), Phenylethylalkohol und Cyclohexyl-2-ethanol.

Als besonders geeignete tertiäre Alkohole seien genannt: 3-Hydroxy-3,7-dimethyl-octan (Tetrahydrolinalool), 7-Hydroxy-3,7-dimethyl-octanal (Hydroxycitronellal), Phenyldimethylcarbinol und 3-Hydroxy-3,7-dimethyl-1,6-octadien (Linalool).

Zur Durchführung der erfindungsgemäßen Umsetzung geht man mit Vorteil so vor, daß man diese Umsetzung in einem geeigneten Reaktionsgefäß durchführt, welches als wesentliche Bestandteile eine gute Rühr- und/oder Mischeinrichtung, eine Dosiervorrichtung für Keten, eine Heizvorrichtung zum Starten der Reaktion und zum Aufrechterhalten der Reaktionstemperatur während der Nachreaktion, eine Kühleinrichtung zum Abführen der Reaktionswärme der exothermen Umsetzung sowie eine Vakuumpumpe enthält. Wenn man jedoch das Keten in das Reaktionsgefäß eindüst, dann ist keine mechanische Durchmischung des Reaktionsgemisches mehr notwendig.

Für eine optimale Reaktionsführung ist wesentlich, daß man das Keten so zudosiert, daß es im Reaktionsgemisch nie im Überschuß vorliegt und daß das Reaktionsgemisch immer gut durchmischt wird.

Bisher haben Acetylierungen mit Keten in der Riechstoffindustrie noch keine große Bedeutung erlangt. Vermutlich haben schlechte Erfahrungen mit Keten dabei eine Rolle gespielt, da Überdosierungen von Keten starke Verfärbungen und Verharzungen des Reaktionsgemisches zur Folge haben.

Für die vorteilhafte Durchführung des erfindungsgemäßen Verfahrens ist es daher wichtig, daß man zu schnelles Zudosieren von Keten vermeidet sowie das Ende der Umsetzung eindeutig feststellt.

Mit Hilfe des erfindungsgemäßen Verfahrens ist es möglich, das in der Riechstoffindustrie begehrte Linalylacetat und zahlreiche andere als Riechstoffe oder Wirkstoffe interessante Essigsäureester von Alkoholen oder Phenolen, wie α-Tocopherol, auf technisch relativ einfache Weise in hohen Reinheiten und dennoch in ausgezeichneten Ausbeuten und Raum-Zeit-Ausbeuten herzustellen.

### Beispiel 1 und Vergleichsbeispiele 1 bis 11

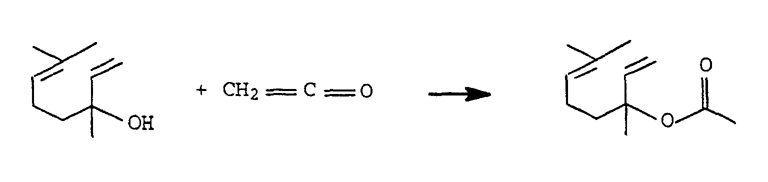

Jeweils 616 g (4,0 mol) Linalool wurden in einem Rührbehälter unter Stickstoffschutz und unter intensivem Rühren (500 U/min) mit einem Scheibenrührer mit 1,23 g Zinkacetat bzw. jeweils 0,2 Gew.-%, bezogen auf Linalool, des aus Tabelle 1 ersichtlichen Katalysators versetzt und unter intensivem Rühren und Solekühlung bei Temperaturen zwischen 80 und 95°C und einem Druck von ca. 500 mbar ca. 80 bis 100 g pro Stunde frisch hergestelltes Keten zudosiert. Die Umsetzung wurde jeweils abgebrochen, wenn von dem Reaktionsgemisch kein Keten mehr aufgenommen wurde.

Das erhaltene Reaktionsgemisch wurde gaschromatographisch untersucht.

In der folgenden Tabelle 1 werden die verwendeten Katalysatoren, die Reaktionszeiten sowie die gaschromatographisch ermittelte Zusammensetzung des Reaktionsgemisches angegeben.

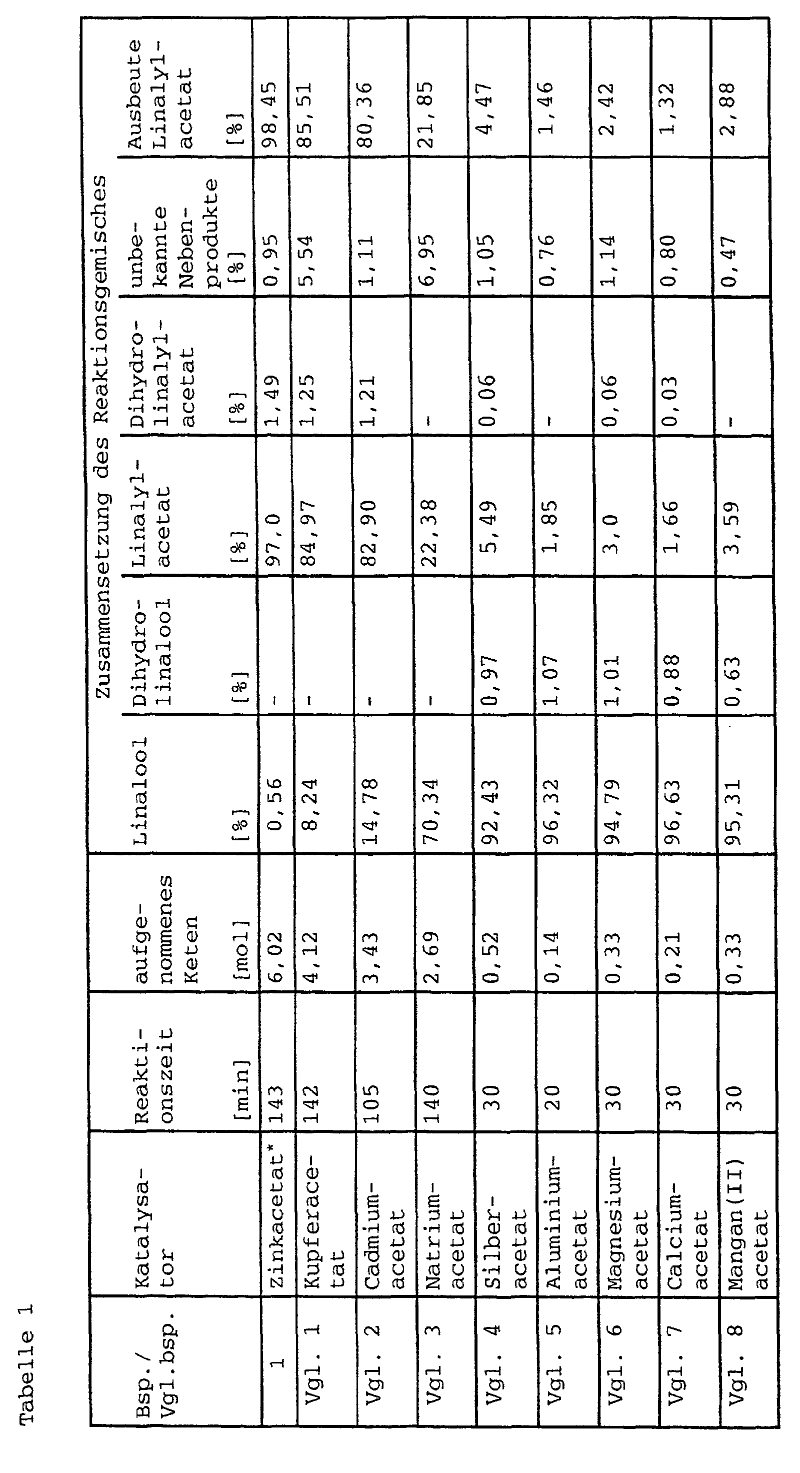

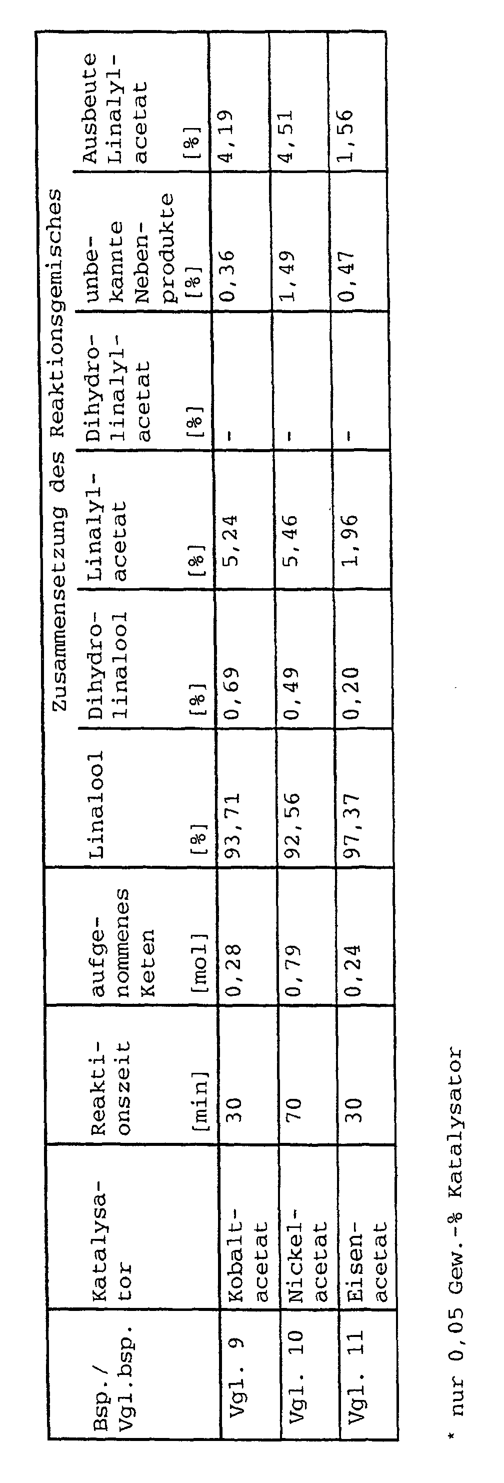

### Beispiele 2 und 3 und Vergleichsbeispiele 12 und 13

Jeweils 616 g (4,0 mol) Linalool wurden in einem Rührbehälter unter Stickstoffschutz und unter intensivem Rühren (500 U/min) mit einem Scheibenrührer mit jeweils etwa 0,05 Gew.-%, bezogen auf Linalool, der aus Tabelle 2 ersichtlichen Zink-Verbindung versetzt und unter intensivem Rühren und Solekühlung bei einer Temperatur von etwa 90°C und einem Druck von ca. 500 mbar 80 bis 100 g/h frisch bereitetes Keten zudosiert.

Die Umsetzung wurde jeweils abgebrochen, wenn von dem Reaktionsgemisch kein Keten mehr aufgenommen wurde.

Das erhaltene Reaktionsgemisch wurde gaschromatographisch untersucht.

In der folgenden Tabelle 2 werden die verwendeten Zink-Verbindungen, die Reaktionszeiten sowie die gaschromatographisch ermittelte Zusammensetzung des Reaktionsgemisches angegeben.

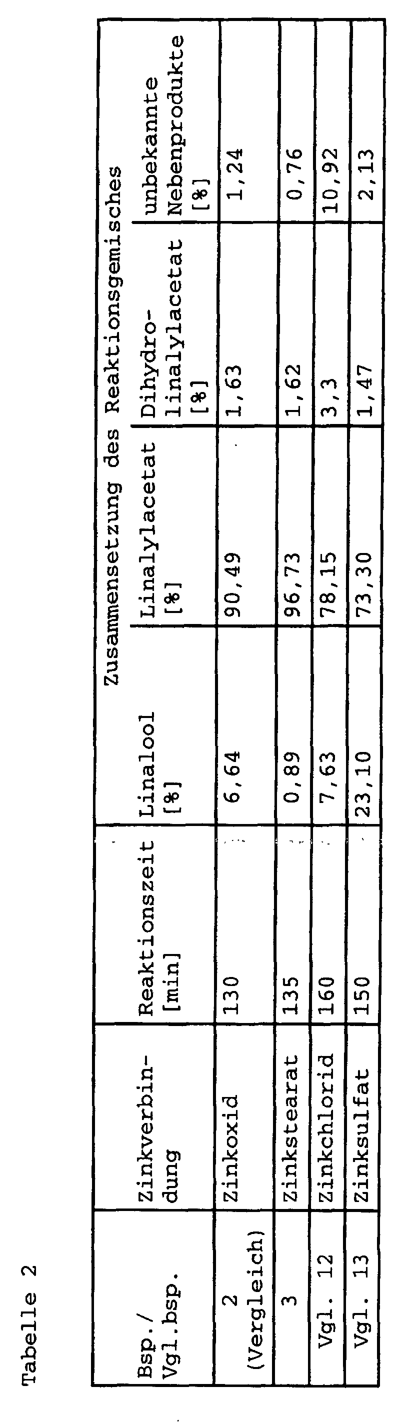

### Beispiel 4

In einem Rührkessel wurden 1305 kg (8474 mol) Linalool unter Stickstoffatmosphäre vorgelegt, hierzu wurden 0,6 kg (0,05 Gew.-%, bezogen auf eingesetztes Linalool) Zinkacetat zugegeben, dann unter intensivem Rühren im Rührkessel der Druck auf 500 mbar verringert und der Reaktorinhalt auf 85°C erhitzt. Anschließend wurde unter Aufrechterhalten einer Reaktionstemperatur von etwa 90°C mittels einer Solekühlung innerhalb von 3 Stunden 500 kg Keten zudosiert. Anschließend wurde das Reaktionsgemisch mit Stickstoff wieder auf Normaldruck gebracht und dann unter Stickstoffschutz noch ca. 30 Minuten bei 90°C nachreagieren lassen.

Das so erhaltene Roh-Linalool wurde gaschromatographisch untersucht.

Es enthielt
6,64 Gew.-% unumgesetztes Linalool
90,49 Gew.-% Linalylacetat und
1,24 Gew.-% Nebenprodukte.

Die Selektivität der Umsetzung betrug demnach 90,26 % der Theorie, bezogen auf umgesetztes Linalool.

### Beispiele 5 bis 10

### Umsetzung mit tertiären Alkoholen und α-Tocopherol

Jeweils die in Tabelle 3 angegebene Menge des in Tabelle 3 angegebenen Alkohols wurde in einem Rührbehälter unter Stickstoffschutz und unter intensivem Rühren mit einem Scheibenrührer mit den aus Tabelle 3 ersichtlichen Mengen an Zinkacetat versetzt und hierzu unter intensivem Rühren und Solekühlung bei den in Tabelle 3 angegebenen Temperaturen und Drucken die aus Tabelle 3 ersichtliche Menge an frisch hergestelltem Keten in einer Geschwindigkeit von ca. 80 bis 100 g pro Stunde zudosiert. Die Umsetzung wurde jeweils abgebrochen, wenn kein Keten mehr aufgenommen wurde.

Die erhaltenen Mengen an Essigsäureestern sowie Ausbeuteangaben können ebenfalls aus Tabelle 3 ersehen werden.

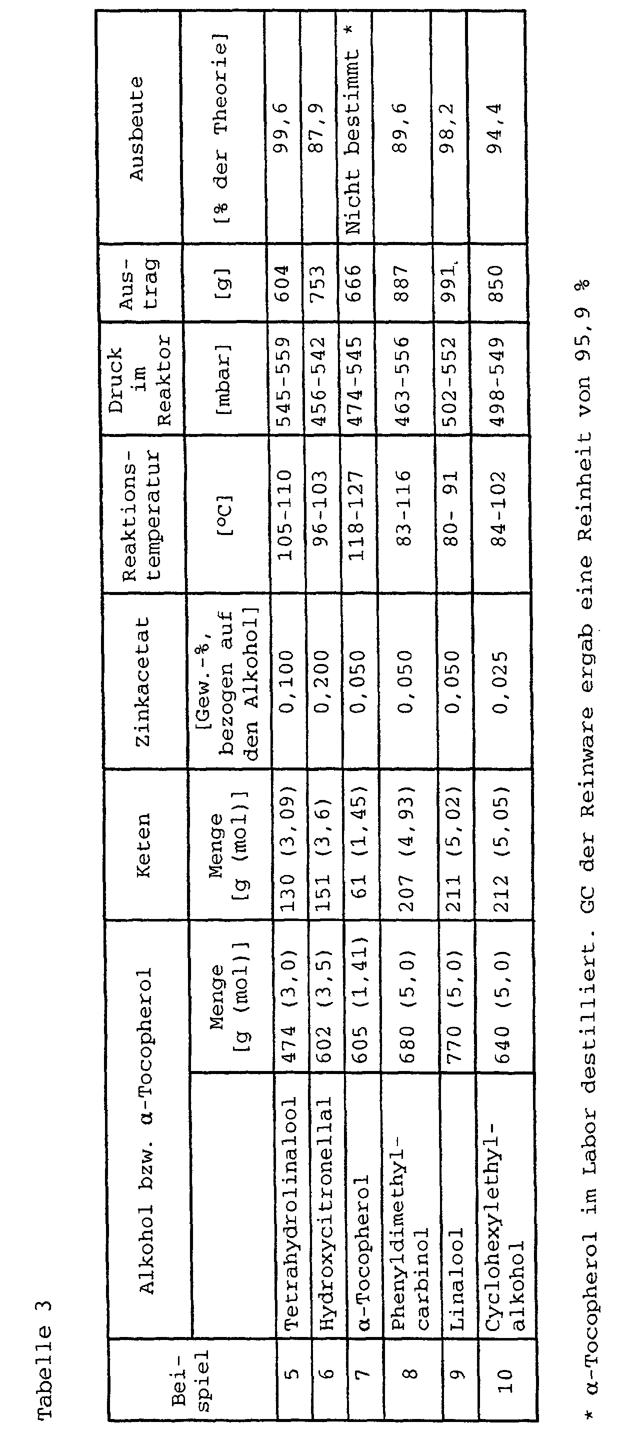

### Beispiele 11 bis 15

### Umsetzung mit primären Alkoholen

Jeweils die in Tabelle 4 angegebene Menge des in Tabelle 4 angegebenen Alkohols wurde in einem Rührbehälter unter Stickstoffschutz und unter intensivem Rühren mit einem Scheibenrührer mit den aus Tabelle 4 ersichtlichen Mengen an Zinkacetat versetzt und hierzu unter intensivem Rühren und Solekühlung bei den in Tabelle 4 angegebenen Temperaturen und Drucken die aus Tabelle 4 ersichtliche Menge an frisch hergestelltem Keten in einer Geschwindigkeit von ca. 80 bis 100 g pro Stunde zudosiert. Die Umsetzung wurde jeweils abgebrochen, wenn kein Keten mehr aufgenommen wurde.

Die erhaltenen Mengen an Essigsäureestern sowie Ausbeuteangaben können aus Tabelle 4 ersehen werden.

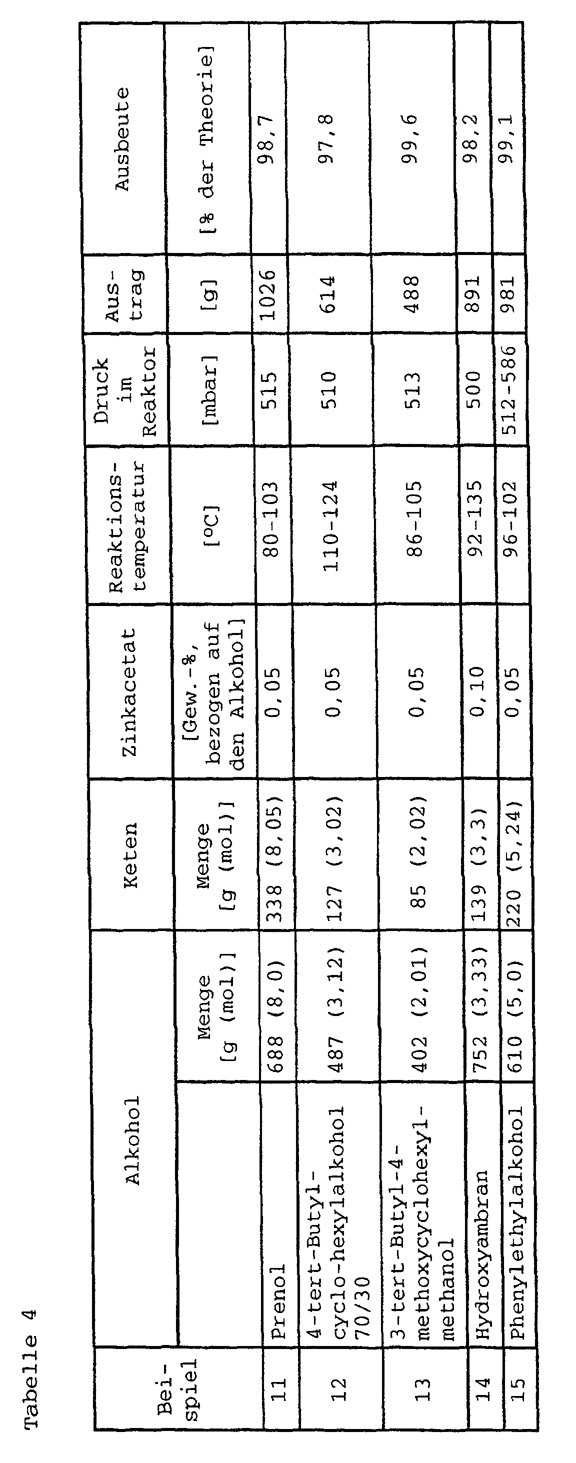

## Patentansprüche

1. Verfahren zur Herstellung von Essigsäureestern durch Umsetzen von eine oder mehrere Hydroxylgruppen enthaltenden organischen Verbindungen mit Keten in Gegenwart von Katalysatoren, **dadurch gekennzeichnet, daß** man die Umsetzung unter Verwendung von etwa der Anzahl der Hydroxylgruppen in der Hydroxylgruppen enthaltenden Verbindung entsprechenden molaren Mengen an Keten und unter intensiver Vermischung des Reaktionsgemisches durchführt und dass man als Katalysatoren Zinksalze von C₂-C₁₈-Carbonsäuren einsetzt.

2. Verfahren gemäß Anspruch 1, **dadurch gekennzeichnet, daß** man die Umsetzung in Gegenwart von Zinkacetat oder von Zinkstearat durchführt.

3. Verfahren gemäß Anspruch 1, **dadurch gekennzeichnet, daß** man als eine oder mehrere Hydroxylgruppen enthaltende organische Verbindung einen gesättigten oder ungesättigten aliphatischen, aromatisch-aliphatischen oder cycloaliphatisch-aliphatischen einwertigen Alkohol mit 5 bis 20 C-Atomen verwendet und diesen mit Keten zu dem entsprechenden Essigsäureester umsetzt.

4. Verfahren gemäß Anspruch 1, **dadurch gekennzeichnet, daß** man als eine oder mehrere Hydroxylgruppen enthaltende organische Verbindung einen tertiären gesättigten oder ungesättigten aliphatischen, aromatisch-aliphatischen oder cycloaliphatisch-aliphatischen Alkohol mit 5 bis 20 C-Atomen verwendet und diesen mit Keten zu dem entsprechenden Essigsäureester umsetzt.

5. Verfahren gemäß Anspruch 1, **dadurch gekennzeichnet, daß** man zur Herstellung von Linalylacetat die Umsetzung unter Verwendung etwa äquimolarer Mengen an Linalool und Keten durchführt.

6. Verfahren gemäß Anspruch 1, **dadurch gekennzeichnet, daß** man die Umsetzung in einem Reaktionsgefäß mit einer guten Rühroder Mischeinrichtung, einer Dosiervorrichtung für Keten, einer Heizquelle zum Starten der Reaktion und für die Nachreaktion, einer Kühleinrichtung zum Entfernen der Reaktionswärme und einer Vakuumpumpe durchführt.

7. Verfahren gemäß Anspruch 1, **dadurch gekennzeichnet, daß** man die Umsetzung bei Temperaturen von 35 bis 110°C und Drucken von etwa 400 bis 900 mbar durchführt.

8. Verfahren gemäß Anspruch 1, **dadurch gekennzeichnet, daß** man Umsetzung bei Temperaturen von 70 bis 100°C durchführt.

9. Verfahren gemäß Anspruch 1, **dadurch gekennzeichnet, daß** man die Umsetzung bei Drücken von etwa 500 bis 600 mbar durchführt.

10. Verfahren gemäß Anspruch 1, **dadurch gekennzeichnet, daß** man Keten verwendet, welches unmittelbar vor der erfindungsgemäßen Umsetzung durch thermische Spaltung von Essigsäure bei Temperaturen von 800 bis 900, vorzugsweise etwa 850°C, und Drucken von 700-900, vorzugsweise 750 bis 850, mbar erhalten worden ist.

## Claims

1. A process for the preparation of acetates by the reaction of organic compounds containing one or more hydroxyl groups with ketene in the presence of catalysts, wherein the reaction is carried out using a molar amount of ketene which approximately corresponds to the number of hydroxyl groups in the hydroxyl group-containing compound, and with vigorous mixing of the reaction mixture, and wherein zinc salts of C₂-C₁₈ carboxylic acids are used as catalysts.

2. A process as claimed in claim 1, wherein the reaction is carried out in the presence of zinc acetate or zinc stearate.

3. A process as claimed in claim 1, wherein the organic compound containing one or more hydroxyl groups used is a monohydric saturated or unsaturated, aliphatic, aromatic/aliphatic or cycloaliphatic/aliphatic alcohol containing from 5 to 20 carbon atoms, which is caused to react with ketene to form the corresponding acetate.

4. A process as claimed in claim 1, wherein the organic compound containing one or more hydroxyl groups used is a tertiary saturated or unsaturated, aliphatic, aromatic/aliphatic or cycloaliphatic/aliphatic alcohol containing from 5 to 20 carbon atoms, which is caused to react with ketene to form the corresponding acetate.

5. A process as claimed in claim 1, wherein the reaction is carried out using approximately equimolar amounts of linalool and ketene for the production of linalyl acetate.

6. A process as claimed in claim 1, wherein the reaction is carried out in a reaction vessel comprising a good stirring or mixing unit, a dosing mechanism for ketene, a source of heat for initiating the reaction and for the subsequent reaction, cooling means for the removal of the heat of reaction and a vacuum pump.

7. A process as claimed in claim 1, wherein the reaction is carried out at temperatures ranging from 35 to 110°C and pressures ranging from approximately 400 to 900 mbar.

8. A process as claimed in claim 1, wherein the reaction is carried out at temperatures ranging from 70 to 100°C.

9. A process as claimed in claim 1, wherein the reaction is carried out at pressures ranging from approximately 500 to 600 mbar.

10. A process as claimed in claim 1, wherein ketene is used which has been obtained by thermal decomposition of acetic acid at temperatures ranging fom 800 to 900, preferably approximately 850, °C and pressures ranging from 700 to 900, preferably 750 to 850, mbar, just prior to the reaction.

## Revendications

1. Procédé pour la préparation d'esters d'acide acétique par réaction d'un ou plusieurs composés organiques contenant des groupes hydroxyle avec un cétène en présence de catalyseurs **caractérisé par le fait qu'**on effectue la réaction en utilisant des quantités molaires de cétène correspondant approximativement au nombre de groupes hydroxyle dans le composé contenant des groupes hydroxyle et sous brassage intensif du mélange réactionnel, et qu'on utilise comme catalyseurs des sels de zinc d'acides de carbone en C₂-C₁₈.

2. Procédé selon la revendication 1, **caractérisé par le fait qu'**on effectue la réaction en présence d'acétate de zinc ou de stéarate de zinc.

3. Procédé selon la revendication 1, **caractérisé par le fait qu'**on utilise comme composé organique contenant un ou plusieurs groupes hydroxyle un alcool monovalent aliphatique, aromatique-aliphatique ou cycloaliphatique-aliphatique, saturé ou insaturé, ayant 5 à 20 atomes de carbone et on le fait réagir avec du cétène pour former l'ester d'acide acétique correspondant.

4. Procédé selon la revendication 1, **caractérisé par le fait qu'**on utilise comme composé organique contenant un ou plusieurs groupes hydroxyle un alcool tertiaire aliphatique, aromatique-aliphatique ou cyclo-aliphatique-aliphatique, saturé ou insaturé, ayant 5 à 20 atomes de carbone et on le fait réagir avec du cétène pour former l'ester d'acide acétique correspondant.

5. Procédé selon la revendication 1, **caractérisé par le fait que**, pour la préparation d'acétate de linalyle, on conduit la réaction en utilisant des quantités approximativement équimolaires de linalol et de cétène.

6. Procédé selon la revendication 1, **caractérisé par le fait qu'**on conduit la réaction dans un réacteur muni d'une bonne installation d'agitation ou de mélange, d'un dispositif de dosage pour le cétène, d'une source de chaleur pour le démarrage de la réaction et pour la post-réaction, d'un dispositif de refroidissement pour évacuer la chaleur de réaction et d'une pompe à vide.

7. Procédé selon la revendication 1, **caractérisé par le fait qu'**on conduit la réaction à des températures de 35 à 110°C et des pressions d'environ 400 à 900 mbar.

8. Procédé selon la revendication 1, **caractérisé par le fait qu'**on conduit la réaction à des températures de 70 à 100°C.

9. Procédé selon la revendication 1, **caractérisé par le fait qu'**on conduit la réaction à des pressions d'environ 500 à 600 mbar.

10. Procédé selon la revendication 1, **caractérisé par le fait qu'**on utilise du cétène qui a été obtenu directement avant la réaction selon l'invention par séparation thermique d'acide acétique à des températures de 800 à 900, de préférence d'environ 850°C, et sous des pressions de 700-900, de préférence de 750 à 850 mbar.
